(19) Europäisches Patentamt
European Patent Office
Office européen des brevets

(11) Numéro de publication: **0 151 052**
**B1**

# (12) FASCICULE DE BREVET EUROPEEN

(45) Date de publication du fascicule du brevet: **09.03.88**

(51) Int. Cl.⁴: **C 07 D 233/61**, A 61 K 31/395

(21) Numéro de dépôt: **85400003.1**

(22) Date de dépôt: **03.01.85**

(54) **Aminoéthylimidazole, composition pharmaceutique en contenant et procédé de préparation.**

(30) Priorité: **11.01.84 FR 8400351**

(43) Date de publication de la demande: **07.08.85 Bulletin 85/32**

(45) Mention de la délivrance du brevet: **09.03.88 Bulletin 88/10**

(84) Etats contractants désignés: **AT BE CH DE FR GB IT LI LU NL SE**

(56) Documents cités:
**EP - A - 0 061 798**
**EP - A - 0 062 918**
**DE - A - 2 407 143**

**Le dossier contient des informations techniques présentées postérieurement au dépôt de la demande et ne figurant pas dans le présent fascicule.**

(73) Titulaire: **JOUVEINAL S.A., Tour Maine Montparnasse 33 Avenue du Maine, F-75755 Paris Cedex 15 (FR)**

(72) Inventeur: **Aubard, Gilbert Gustave, 7 Chemin de la Savetière, F-91120 Palaiseau (FR)**
Inventeur: **Bure, Jacques, 22, rue Perronet, F-92200 Neuilly Sur Seine (FR)**
Inventeur: **Grouhel, Agnès Geneviève, 2, rue des Peupliers, F-92190 Meudon (FR)**
Inventeur: **Junien, Jean-Louis, 85, Boulevard Suchet, F-75016 Paris (FR)**
Inventeur: **Lelievre, Véronique Jeanne Marie, 11, rue Barrault, F-75013 Paris (FR)**
Inventeur: **Pascaud, Xavier Bernard, 10, rue des Gravilliers, F-75003 Paris (FR)**
Inventeur: **Roux, Claude Paul Joseph, 32 Square Montsouris, F-75014 Paris (FR)**

(74) Mandataire: **Bourgognon, Jean-Marie et al, Cabinet Flechner 22, Avenue de Friedland, F-75008 Paris (FR)**

EP 0 151 052 B1

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

## Description

La présente invention concerne de nouveaux dérivés de l'imidazole substitués en leur position 1 par un radical 2-N,N-diméthylaminoéthyle, leur procédé de préparation et leur application en médecine humaine et vétérinaire.

Les produits selon l'invention répondent à la structure générale suivante:

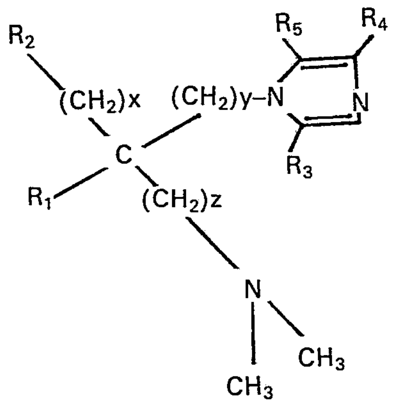

(I)

dans laquelle:

$R_1$ est alcoyle ayant jusqu'à six atomes de carbone.

$R_2$ est soit alcoyle ayant jusqu'à six atomes de carbone, soit un radical phényle ou phénoxy éventuellement substitué par un atome de chlore, par un radical alcoxy ayant jusqu'à six atomes de carbone ou par un radical phényle.

$R_3$, $R_4$ et $R_5$ peuvent être identiques ou différents et sont des atomes d'hydrogène, des radicaux alcoyle ayant jusqu'à six atomes de carbone ou encore des radicaux phényle.

x, y et z ont pour valeur 0 ou 1, les valeurs de y et de z ne pouvant toutefois être égales, et leurs sels d'addition avec des acides.

D'une façon générale, les radicaux alcoyle et alcoxy comprennent de 1 à 6 atomes de carbone en chaîne lineaire ou ramifiée.

Lorsque $R_2$ est un radical phénoxy ou phényle il peut être plurisubstitué ou monosubstitué. Quand il est monosubstitué, la monosubstitution est préférentiellement située en position para par un atome de chlore, ou par un radical alcoxy ayant jusqu'a six atomes de carbone comme le radical méthoxy, ou encore par un radical aromatique comme le radical phényle.

L'invention englobe également les mono ou di sels d'addition de ces dérivés, notamment d'acides minéraux tels que les halohydrates, sulfates, phosphates ou ceux d'acides organiques tels que les maléates, citrates, malates, tartrates, méthanesulfonates, camphosulfonates, benzoates, etc.

Il va de soi que l'invention porte aussi bien sur les formes racémiques des produits que sur leurs formes optiquement actives que l'on peut obtenir par des moyens bien connus, comme par exemple le dédoublement des formes racémiques au moyen d'acides optiquement actifs, ou encore par préparation à partir de structures optiquement actives.

Les études pharmacologiques démontrent que parallèlement à une faible toxicité, les produits de l'invention possèdent une activité cytoprotectrice particulièrement remarquable appropriée au traitement préventif ou curatif des affections du tractus gastrintestinal. L'invention vise donc aussi une composition pharmaceutique comprenant un composé de formule (I) ou un sel de celui-ci associé à un véhicule pharmacologiquement acceptable. Le composé représente en général de 10 à 50 parties en poids de la composition, tandis que le véhicule en représente de 90 à 40 parties en poids.

Les produits de l'invention sont préparés par N-alcoylation en position 1 du radical imidazole approprié au moyen d'halogénures de 2-N,N-diméthylaminoéthyle suivant la réaction:

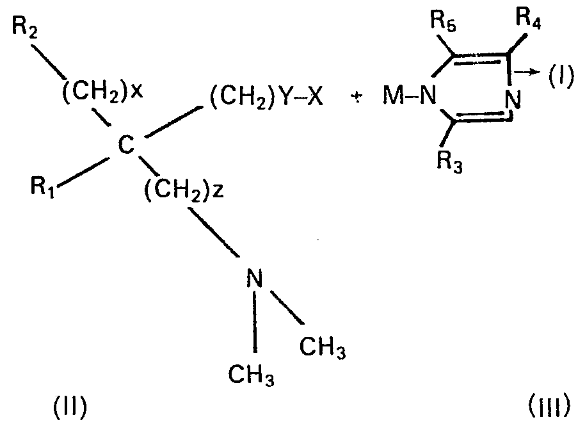

(II) (III)

réactifs (II) et (III) dans lesquels $R_1$, $R_2$, $R_3$, $R_4$ et $R_5$, x, y et z ont les significations précédemment décrites et dans lesquels X est un atome d'halogène tel qu'un atome de chlore et M est un atome d'hydrogène ou un atome métallique tel que l'argent, le sodium ou le potassium. Les halogénures (II) sont accessibles à partir des amino-alcools correspondants comme par exemple par réaction du chlorure de thionyle, comme il est décrit dans «Organic Synthèses», Coll. Vol. IV, page 333 ou par d'autres méthodes appropriées décrites dans la littérature. Les amino-alcools de départ sont eux-mêmes préparés par des méthodes connues, comme par exemple celle décrite aux brevets français Nos. 912 577 et 71 08 700.

Les composés imidazole (III) sont pour certains accessibles dans le commerce ou sont préparés selon des méthodes connues qui consistent à faire réagir sur l'imidazole appropriée des métaux ou leurs hydroxyles, leurs hydrures, leurs alcoolates ou encore leurs amidures. Les dérivés (III) obtenus peuvent être soit isolés, soit préparés in situ de façon préliminaire dans le milieu réactionnel de préparation des produits de l'invention.

La réaction de condensation pour la préparation des produits de l'invention peut s'effectuer dans différents milieux:

– en milieu monophasique dans des solvants anhydres et inertes représentant de 3 à 100 fois et mieux de 8 à 15 fois le poids des deux réactifs comme des hydroacarbures halogénés, tels que le

tétrachlorure de carbone, le dichlorométhane, le chloroforme, des hydrocarbures aromatiques, tels que le benzène, le toluène, des éthers, tels que l'éther diéthylique, des éthers cycliques, tels que le tétrahydrofuranne, le dioxanne, des hydrocarbures aliphatiques saturés éventuellement alcoxylés, tels que le diméthoxyéthane, l'hexane, l'heptane, des cétones telles que l'acétone, la méthyléthylcétone, des sulfoxydes, tels que le diméthylsulfoxyde, des amides tels que le diméthylformamide, le diméthylacétamide, l'hexaméthylphosphorotriamide, etc.,

– en milieu biphasique en présence d'eau et d'un solvant aromatique comme le benzène ou le toluène. La réaction s'effectue alors en milieu très alcalin en présence d'hydroxyde de sodium ou de potassium et en présence d'un catalyseur dit «de transfert de phase» comme des sels d'ammonium quaternaire qui peuvent être des bromures, chlorures, iodures, hydrogénosulfates ou tétrafluoroborates de tétraméthylammonium, de triéthylbenzylammonium, de triméthyloctadécylammonium, ou encore des dérivés phosphonium comme le bromure de tributylhexadécylphosphonium. L'eau représente de 10 à 30 parties en volume pour 100 parties en volume de phase organique. L'ensemble eau et solvant organique représente de 3 à 100 fois et mieux de 8 à 15 fois du poids des deux réactifs. Le catalyseur représente 0,5 à 5 parties du poids des deux réactifs.

Le plus souvent, on utilise 0,5 à 2 moles de dérivé (III) par mole de dérivé (II)et plus favorablement un rapport équimolaire entre ces deux réactifs.

Les réactions s'effectuent à des températures comprises entre 20° et 150 °C selon le type de réaction et les solvants utilisés. On préfère cependant utiliser la réaction de condensation selon la méthode dite «par transfert de phase» à une température comprise entre 50 et 100 °C.

La durée des réactions est comprise entre 1 et 10 heures et de façon préférée entre 2 et 5 heures.

Les produits de l'invention sont isolés du milieu réactionnel par des procédés connus comme l'extraction, la distillation, la cristallisation et la chromatographie séparative.Leur identité et leur pureté sont vérifiée par des moyens conventionnels comme les méthodes spectrographiques (U.V., I.R., R.M.N., masse), chromatographiques (C.C.M., C.L.H.P.) ou physico- chimiques (point de fusion F, indice de réfraction $n_D^{20}$).

Les exemples qui suivent décrivent de façon non limitative les produits de l'invention.

Exemple 1

<u>1A</u>: 1-[(2-phényl 2-éthyl)2-N,N-diméthylaminoéthyl]-imidazole

$R_1$ = éthyle, $R_2$ = phényle, $R_3$ = $R_4$ = $R_5$ = hydrogène,

x = z = 0,y =1.

<u>1B</u>: 1-[(1-phényl 1-éthyl)2-N,N-diméthylaminoéthyl]-imidazole

$R_1$ = éthyle, $R_2$ = phényle, $R_3$ = $R_4$ = $R_5$ = hydrogène,

x = y = 0, z =1.

Dans un réacteur d'un litre sous atmosphère d'zote, on introduit 100 ml de diméthylformamide (DMF) anhydre et 7,1 g d'hydrure de sodium à 80% (p/p) (5,66 g–0,236 mole).

A la suspension grisâtre, on ajoute sous agitation une solution de 16,0 g (0,236 mole) d'imidazole dans 100 ml de DMF en 15 minutes environ et à une température inférieure à 25 °C. On obtient une solution verdâtre qui est maintenue 20 minutes sous agitation à température ambiante.

On introduit ensuite en 20 minutes à une température voisine de 20 °C 50,0 g (0,236 mole) de chlorure de 2-phényl 2-éthyl 2-N,N-diméthylaminoéthyle en solution dans 100 ml de DMF.

Le mélange est agité durant 2 heures 30 à température ambiante, puis précipité dans 1000 ml d'eau glacée et extrait à l'éther. Les phases éthérées réunies sont lavées à l'eau jusqu'à neutralité puis séchées sur sulfate de sodium.

L'éther est éliminé par distillation sous vide et sur bain-marie. On obtient un résidu huileux (46 g) qui est traité pour obtenir les produits IA et IB.

<u>1A</u>. Le résidu est repris par 400 ml de cyclohexane à ébullition. La solution abandonnée à la température ordinaire cristallise. Les cristaux sont filtrés et séchés à 60 °C sous vide. Poids: 19,0 g – Rendement = 33,1%. F = 91 °C.

1A, Hémimaléate.

13,25 g (0,054 mole) du produit précédent sont salifiés par 6,6 g (0,057 mole) d'acide maléique dans 130,0 ml d'éthanol au reflux. On obtient 17,6 g de cristaux blancs. Rendement = 90,7%. F = 124 °C.

1A, Iodométhylate.

14,0 g (0,0575 mole) de produit sont dissous dans 85,0 ml d'acétone anhydre. On ajoute rapidement 24,5 g (0,173 mole) d'iodure de méthyle et on laisse agiter une nuit à température ambiante. On obtient 20,2 g de cristaux jaune pâle. Rendement = 91,2%. F = 163 °C.

<u>1B</u>. Le cyclohexane de cristallisation de la base du produit 1A est éliminé par distillation sous vide. La résidu obtenu (28,0 g est purifié par chromatographie sur colonne de silice. L'élution par un mélange cyclohexane-acétate d'éthyle-méthanol 12-3-1 (v/v/v) permet de recueillir une fraction pure en chromatographie sur couche mince. Poids: 8,7 g Rendement = 15,2%. $n_D^{20}$ = 1,5471.

La spectrographie de résonance magnétique du proton et la spectrographie de masse des produits sont en accord et permettent d'attribuer sans équivoque les structures annoncées pour les produits 1A et 1B.

Exemple 2

<u>2A</u>: 1[(2-phényl 2-éthyl)2-N,N- diméthylaminoéthyl]-2méthylimidazole

$R_1$: éthyle, $R_2$ = phényle, $R_3$ = méthyle, $R_4$ = $R_5$ = hydrogène

x, z = 0, y = 1.

<u>2B</u>: 1-[(1-phényl 1-éthyl)2-N,N-diméthylaminoéthyl]- 2-méthylimidazole

$R_1$ = éthyle, $R_2$ = phényle, $R_3$ = méthyle, $R_4$ = $R_5$ = hydrogène,
x = y = 0, z = 1.

Dans un réacteur d'un litre, on introduit 300,0 ml de toluène, 11,6 g (0,142 mole) de 2-méthylimidazole, 30,0 g (0,142 mole) de chlorure de 2-phényl 2-éthyl 2-N,N-diméthylaminoéthyle, puis 45,0 ml de solution aqueuse d'hydroxyde de sodium (d = 1,33) et 2,7 g de bromure d'hexadécytributylphosphonium.

Sous agitation énergique, le mélange est porté 2 heures à 80–85 °C. Après refroidissement, la phase aqueuse est éliminée et la phase toluénique lavée par extraction avec une solution saturée de chlorure de sodium.

La phase toluénique est séchée sur sulfate de sodium puis le toluène est éliminé par distillation sous vide et sur le bain-marie.

Le résidu huileux (38,5 g) est traité de façon identique à l'exemple 1 pour séparer les produits 2A et 2B. Après traitements et purifications, on obtient:

2A, Hémimaléate:
Poids = 17,0 g. Rendement = 24,5%. F = 111,5 °C.
2B: Poids = 7,0 g. Rendement = 19,2%. F = 67,5 °C.

Les méthodes spectrographiques utilisées confirment sans équivoque les structures des produits 2A et 2B.

Les produits des exemples 3 à 8 sont décrits dans le tableau 1 qui suit. Ils sont préparés selon le mode opératoire de l'exemple 2 à partir des produits de formule générale II et III dans lesquels les significations de $R_1$, $R_2$, $R_3$, $R_4$, $R_5$, x, y et z sont définies.

Pour les produits de formule II, X est un atome de chlore alors que pour les produits de formule III, M est un atome d'hydrogène ou de sodium.

Tableau 1

| Ex. No. | $R_1$ | $R_2$ | $R_3$ | $R_4$ | $R_5$ | x | A y = 1 z = 0 | B y = 0 z = 1 |
|---|---|---|---|---|---|---|---|---|
| 3 | $C_2H_5$ | 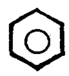 | H | 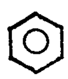 | 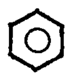 | 0 | Base – F = 183 °C<br>Rendement = 19,3%<br>H. maléate F = 142 °C | Base F = 133 °C<br>Rendement = 31,4% |
| 4 | $C_2H_5$ | 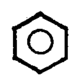 | $C_2H_5$ | $CH_3$ | H | 0 | Base – F = 56 °C<br>Rendement = 12,2% | Base – Huile<br>Rendement = 14,75% |
| 5 | $C_2H_5$ | 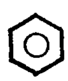 | 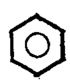 | H | H | 0 | Base – Huile<br>Rendement = 7%<br>H. maléate F = 135 °C | Base F = 80,5 °C<br>Rendement = 14,2% |
| 6 | $CH_3$ | $CH_3$ | H | H | H | 0 | Base – Huile $n_D^{20}$ = 1,501<br>Rendement = 19% | Base – Huile $n_D^{20}$ = 1,469<br>Rendement = 15% |
| 7 | $C_2H_5$ | 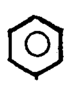 | H | H | H | 1 | Base – Huile<br>Rendement = 27,9%<br>Dicamphosulfonate<br>F = 128 °C | Base F = 60 °C<br>Rendement = 3,7% |
| 8 | $C_5H_{11}$ | 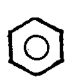 | H | H | H | 0 | Base – Huile $n_D^{20}$ = 1,542<br>Rendement = 27,5% | Base – Huile $n_D^{20}$ = 1,532<br>Rendement = 17,6% |

Exemple 9

1-[(2-p méthoxybenzyl 2-méthyl)-N,N-diméthylaminoéthyl]imidazole, dihémimaléate.

$R_1$ = méthyle, $R_2$ = p.méthoxybenzyle, $R_3$ = $R_4$ = $R_5$ = hydrogène, x = 1, z = 0, y = 1.

Dans un réacteur de 500 ml, on introduit 200 ml de toluène, 5,5 g (0,081 mole) d'imidazole, 19,6 g (0,081 mole de chlorure de 2-(p-méthoxybenzyl)- 2-méthyl-2-N,N diméthylaminoéthyle, 32,6 ml de solution aqueuse d'hydroxyde de sodium (d = 1,33) et 2,0 g de bromure d'hexadécyl-tributylphosphonium.

Le mélange est porté 4 heures à 85–90° sous agitation énergique puis, après refroidissement, la phase aqueuse est écartée.

La phase toluénique est lavée par une solution saturée de chlorure de sodium, déshydratée, puis le toluène est éliminé par distillation. On obtient un résidu de 22,0 g qui est purifié par chromatographie sur colonne de silice.

L'élution par un mélange cyclohexane-acétate d'éthyle-méthanol 100-40-15 (v/v/v) permet d'isoler le produit désiré. Poids = 9,8 g. Rendement = 46,1%.

8,4 g (0,032 mole) de produit sont salifiés par 7,75 g (0,067 mole) d'acide maléique dans 150 ml d'éthanol au reflux. Après refroidissement, les cristaux obtenus sont filtrés. Poids = 10,0 g. Rendement = 61,8% F= 122,5 °C.

A partir des réactifs appropriés et selon le mode opératoire décrit pour l'exemple 9, on obtient les produits des exemples 10 et 11 (tableau 2) dans lesquels $R_3 = R_4 = R_5 = H$, z = 0 et y = 1.

Tableau 2

| Ex. No. | $R_1$ | $R_2$ | X | Produit |
|---|---|---|---|---|
| 10 | $CH_3$ | 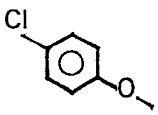 | 1 | Base $n_D^{20} = 1{,}5540$ Rendement = 34% |
| 11 | $CH_3$ | 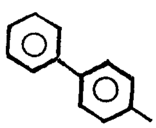 | 0 | Base F = 125,1 °C Rendement = 15% |

Exemple 12

1-[(2-phényl 2-éthyl) 2-N,N-diméthylaminoéthyl]-imidazole.

Le dérivé chloré correspondant à l'isomère lévogyre 2-phényl 2-éthyl N,N-diméthylaminoéthanol est préparé selon la technique habituelle.

Le produit obtenu est condensé avec l'imidazole selon la technique décrite dans l'exemple 2.

Après traitement et purification, on obtient 18,8 g de produit huileux. Rendement = 38,6%. $[\alpha]_D^{20} = /-27{,}8°$ (EtOH c = 1%).

Hémimaléate

18 g de produit précédent (0,074 mole) sont salifiés au reflux de l'éthanol par 9 g (0,078 mole) d'acide maléique.

On obtient 20 g de cristaux blancs. Rendement = 75%. F = 125 °C. $[\alpha]_D^{20} = 29{,}2°$ ($H_2O$ c = 2%).

Les essais toxicologiques et pharmacologiques décrits dans la partie qui suit montrent que les produits de l'invention ne sont que peu toxiques et présentent des activités cytoprotectrices intéressantes.

La toxicité aiguë des produits a été étudiée chez la souris SWISS/IOPS mâle d'environ 22 g. La dose létale 50% ($DL_{50}$) a été calculée par la méthode de L.J. Reed et H. Munch. (Am. J. Hyg. 1939–37–493) quatorze jours après traitement par voie orale ou intrapéritonéale de lots de 10 animaux par dose et à partir de 4 à 6 doses par produit. Les résultats de cette étude sont présentés dans le tableau 3 pour les produits des exemples 1 à 8 et dans le tableau 4 pour les produits des exemples 9 à 12.

Tableau 3
Toxicité aigue – Ex. 1 à 8

| Produit codé | | $DL_{50}$ % mg/kg$^{-1}$ | | Produit codé | | $DL_{50}$ % mg/kg$^{-1}$ | |
|---|---|---|---|---|---|---|---|
| | | Voie orale | Voie i.p. | | | Voie orale | Voie i.p. |
| Ex. 1A, | H, maléate JO 1193 | 559 | 326 | Ex. 1B | JO 1194 | 285 | 145 |
| Ex. 2A, | H, maléate JO 1230 | 500 | 157 | Ex. 2B | JO 1231 | 393 | 137 |
| Ex. 3A, | H, maléate JO 1323 | >1150 | | Ex. 3B | JO 1324 | >1152 | |
| | | | | Ex. 4B | JO 1325 | 298 | 47 |
| | | | | Ex. 5B | JO 1334 | 840 | |
| Ex. 6A | JO 1331 | >1180 | | Ex. 6B | JO 1332 | >1180 | |
| Ex. 71, | Di Camphos. JO 1365 | 480 | | Ex. 7B | JO 1364 | 384 | |
| Ex. 8A | JO 1368 | 287 | | Ex. 8B | JO 1367 | 562 | 346 |

Tableau 4
Toxicité aigue – Ex. 9 à 12

| Produit codé | $DL_{50}$ % $mg/kg^{-1}$ Voie orale | Voie i.p. |
|---|---|---|
| Ex. 9, Di H, maléate JO 1250 | 758 | 231 |
| Ex. 10 JO 1233 | 86 | 32 |
| Ex. 11 JO 1366 | 183 | |
| Ex. 12, H. maléate JO 1274 | 780 | |

L'activité cytoprotectrice a été étudiée vis-à-vis de l'agression de la muqueuse gastrique provoquée par l'éthanol.

Le test de cytoprotection a été réalisé selon la technique décrite par A. Robert et al. (Gastroenterology 1979-77-433). Des lots de six rats Sprague Dawley mâles, mis à jeun de nourriture solide 24 heures avant le test, reçoivent par voie orale 1 ml/rat d'éthanol absolu, après avoir reçu le produit à tester 30 minutes auparavant par voie sous-cutanée ou une heure auparavant par voie orale. Une heure après l'ingestion de l'éthanol, les rats sont sacrifiés par élongation et leurs estomacs délicatement prélevés, ouverts selon la grande courbure, lavés sous eau courante et étalés. Les ulcérations sont ensuite repérées et mesurées sous une lampe binoculaire. Un index représentant la somme des longueurs des ulcères est ensuite déterminé pour chaque rat et la moyenne de ces index par lot est calculée et comparée à celle du lot contrôle à l'aide du test «t» de Student. La dose efficace 50% ($DE_{50}$) est ensuite déterminée graphiquement lorsque cela est possible. Dans les autres cas, le pourcentage d'inhibition obtenu à l'aide de la dose maximum testée est présenté. Les résultats sont présentés dans le tableau 5 pour les produits des exemples 1 à 8 et dans le tableau 6 pour les produits des exemples 9 à 12.

Tableau 5
Activité cytoprotectrice – Ex. 1 à 8

| Produit codé | | $DL_{50}$ % $mg/kg^{-1}$ Voie orale | Voie s.c. | Produit codé | | $DL_{50}$ % $mg/kg^{-1}$ Voie orale | Voie s.c. |
|---|---|---|---|---|---|---|---|
| Ex. 1A, | H. maléate JO 1193 | $DE_{50}$ % 13,0 | $DE_{50}$ % 11,0 | Ex. 1B | JO 1194 | $DE_{50}$ % 7,1 | $DE_{50}$ % 8,1 |
| Ex. 2A, | H. maléate JO 1230 | $DE_{50}$ % 12,0 | $DE_{50}$ % 12,6 | Ex. 2B | JO 1231 | $DE_{50}$ % 12,0 | $DE_{50}$ % 10,0 |
| | | | | Ex. 3B | JO 1324 | $DE_{50}$ % 26,0 | |
| | | | | Ex. 4B | JO 1325 | | $DE_{50}$ % 4,0 |
| Ex. 7A, | Di Camphos. JO 1365 | | $DE_{50}$ % 26,6 | Ex. 7B | JO 1364 | | $DE_{50}$ % 8,5 |
| Ex. 8A | JO 1368 | | $DE_{50}$ % 25,0 | Ex. 8B | JO 1367 | | $DE_{50}$ % 9,0 |

Tableau 6
Activité cytoprotectrice – Ex. 9 à 13

| Produit codé | | DL X % $mg/kg^{-1}$ Voie s.c. |
|---|---|---|
| Ex. 10 | JO 1233 | $DE_{50}$ % 10,0 |
| Ex. 11 | JO 1366 | $DE_{40}$ % 10,0 |
| Ex. 12, H. maléate | JO 1274 | $DE_{50}$ % 5,3 |

Les résultats résumés dans les tableaux précédents montrent que les produits de l'invention protègent de manière significative des hémorragies et ulcérations de la muqueuse gastrique provoquées chez le rat par l'absorption d'éthanol absolu. Cette activité se manifeste aussi bien par voie parentérale que par voie orale, les $DE_{50}$ par ces deux voies étant à peu près équivalentes pour les composés testés par ces deux voies (JO 1193, 1194, 1230 et 1231).

Par ailleurs, il paraît particulièrement intéressant de remarquer que cette activité se manifeste à des doses très éloignées des doses toxiques (1/20 ou 1/40 de la $DL_{50}$).

Les produits de l'invention montrent également une activité inhibitrice de la Thromboxane Synthétase. Cette activité peut être reliée aux propriétés cytoprotectrices précédemment décrites.

En effet, en résumant très brièvement des travaux récents, il apparaît que les prostanoïdes jouent un rôle déterminant dans les phénomènes vaso actifs de l'inflammation. Ainsi, dans certains cas d'inflammation ulcéreuse, il a été démontré aux niveaux gastrique et duodénal, une diminution de la synthèse des $PGE_2$ et $PGI_2$, ces produits étant des médiateurs qui possèdent des effets antisécrétoires acides, stimulateurs de la sécrétion alcaline et étant en eux-mêmes cytoprotecteurs.

Inversement, les effets du $TXA_2$ seraient opposés à ceux de $PGI_2$. Le rapport entre ces deux types de prostanoïdes est donc important et d'autant plus favorable avec une augmentation des PG et une diminution des TX. L'inhibition de la Thromboxane Synthétase par les produits de l'invention est donc significative et/ou complémentaire de l'activité cytoprotectrice.

Le test pratiqué a été réalisé «in vitro» sur des plaquettes lavées de lapin. Il a été inspiré des publications de Needleman (1977. Proc. Natl. Acad. Sci. 74.1716) et de Salmon (1982, Cardiovascular Pharmacol. of the Prostaglandins – Raven Pess N.Y. p. 7 à 22). Il permet à la fois de déterminer la diminution de synthèse des $TXB_2$ et d'autre par l'éventuelle augmentation de synthèse des $PGE_2$ – Il est ainsi possible de déterminer l'activité des produits sur le rapport $\frac{PG}{TX}$.

Les résultats présentés dans les tableaux qui suivent sont exprimés en CE x % c'est-à-dire en concentrations micromolaires par litre permettant une inhibition de x % de la synthèse du $TXB_2$. Dans la colonne voisine, la présence d'un signe + signifie que l'effet provoque également une augmentation de synthèse de la $PGE_2$.

Dans ce test, le 1-benzyl imidazole reconnu comme inhibiteur de Thromboxane Synthétase sur les plaquettes humaines (Hsin-Hsiung Tai – Barbara Yuan – Bioch.Biophys. Res. Comm. 1978 1 80 p. 236) a été utilisé comme substance de référence. Certains produits de l'invention montrent une activité égale ou nettement supérieure à ce produit en particulier les produits référencés JO 1193, 1368, 1367 et 1250.

Tableau 7
Resultats des exemples 1 à 8

| Produit codé | | Inh. TX $B_2$ | Augm. $PGE_2$ | Produit ccdé | | Inh. TX $B_2$ Augm. | $PGE_2$ |
|---|---|---|---|---|---|---|---|
| Ex. 1A, | H. maléate JO 1193 | $CE_{50}$% = 3,7 | + | Ex. 1B | JO 1194 | $CE_{50}$% = 17,4 | + |
| Ex. 2A, | H. maléate JO 1230 | $CE_{10}$% = 50 | | Ex. 2B | JO 1231 | $CE_{18}$% = 50 | |
| Ex. 5A | JO 1333 | $CE_{50}$% = 50 | | Ex. 5B | JO 1334 | $CE_{20}$% = 50 | |
| Ex. 6A | JO 1331 | $CE_{41}$% = 50 | | Ex. 6B | JO 1332 | $CE_{50}$% = 50 | |
| Ex. 7A, | Di Camphos JO 1365 | $CE_{50}$% < 1 | + | Ex. 7B | JO 1364 | $CE_{35}$% = 100 | |
| Ex. 8a | JO 1368 | $CE_{50}$% = 0,56 | + | Ex. 8B | JO 1367 | $CE_{50}$% = 2,15 | + |

Tableau 8
Resultats des exemples 9 à 12

| Produit codé | Inh. $TXB_2$ | Augm. $PGE_2$ |
|---|---|---|
| Ex. 9, Di H. maléate | $CE_{50}$ % = 4,5 | + |
| Ex. 10 JO 1233 | $CE_{50}$ % = 13,2 | + |
| Ex. 11 JO 1366 | $CE_{50}$ % = 11,2 | + |
| Ex. 12, H. maléate JO 1274 | $CE_{50}$ % = 11,3 | + |
| Réf. 1-benzyl imidazole | $CE_{50}$ % = 13,4 | |

Les produits de l'invention sont administrés à l'être humain par des voies appropriées à la nature et à la gravité de l'affection à traiter et sous des formes thérapeutiques compatibles avec la voie d'administration envisagée. Ces différentes formes préparées à partir des produits de l'invention sous forme de base ou de leurs différents sels sont préparées par des méthodes connues en soi.

Les différentes préparations peuvent également contenir les produits de l'invention en association avec des principes actifs compatibles tels que des bactériostatiques, antibiotiques, antispasmodiques, anesthésiques, analgésiques, antiinflam-

matoires, etc. Pour exemples de ces préparations, on citera les comprimés, dragées, capsules, poudres, solutions, suspensions, gels et suppositoires et pour illustrer de façon non limitative leur fabrication, on donne ci-dessous celle des comprimés et celle des solutés isotoniques injectables avec les principes actifs de l'invention.

| 1) Comprimés | mg |
|---|---|
| Substance active selon l'exemple 2A | 50,0 |
| Lactose | 26,5 |
| Mannitol | 55,0 |
| Sucre blanc officinal | 11,0 |
| Polyéthylène glycol 6000 | 05,0 |
| Stéarate de magnésium | 02,0 |
| Gélatine | 00,5 |
| Amidon de blé | 50,0 |
| Total | 200,0 |

Séparément, la gélatine et le surcre blanc officinal sont dissous dans l'eau. Les deux solutions sont mélangées, on y ajoute le polyéthylène glycol 6000.

Par ailleurs, le lactose et le mannitol sont mélangés intimement puis on ajoute, dans l'ordre, la substance active de l'exemple 2A, l'amidon de blé, puis la solution obtenue précédemment.

La pâte est séchée, granulée et tamisée en y ajoutant le stéarate de magnésium et l'amidon de blé.

Le produit obtenu est homogénéisé et compressé à raison de 200,0 mg par comprimé.

| 2) Solution isotonique injectable | mg |
|---|---|
| Substance active selon l'exemple 1A | 10 |
| Chlorure de sodium | 9 |
| Eau distillée (quantité suffisante pour) | 1 ml |

La soluté isotonique est réparti en ampoules de volume approprié qui sont, après scellement, stérilisées par des moyens thermiques connus en soi, ou bien le soluté est stérilisé par filtration, réparti en ampoules qui sont ensuite scellées, l'ensemble des opérations étant effectué sous atmosphère stérile.

Dans ce dernier cas, on préfère ajouter à la formule décrite, 1% d'alcool benzylique à titre d'agent bactériostatique, soit 10 mg de cet alcool par ml de soluté.

**Revendications pour les Etats contractants BE, CH, DE, FR, GB, IT, LI, LU, NL, SE**

1. Aminoéthylimidazole de formule:

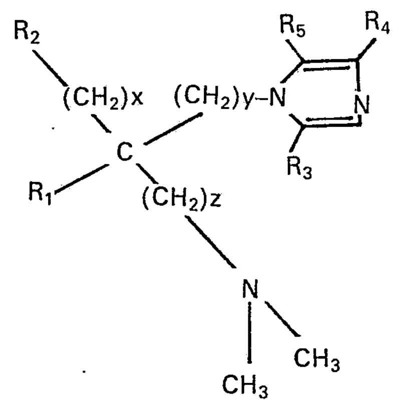

(I)

dans laquelle:

$R_1$ est alcoyle ayant jusqu'à six atomes de carbone.

$R_2$ est soit alcoyle ayant jusqu'à six atomes de carbone, soit un radical phényle ou phénoxy éventuellement substitué par un atome de chlore, par un radical alcoxy ayant jusqu'à six atomes de carbone ou par un radical phényle.

$R_3$, $R_4$ et $R_5$ peuvent être identiques ou différents et sont des atomes d'hydrogène, des radicaux alcoyle ayant jusqu'à six atomes de carbone ou encore des radicaux phényle.

x, y et z ont pour valeur 0 ou 1, les valeurs de y et de z ne pouvant toutefois être égales, et leurs sels d'addition avec des acides.

2. Aminoéthylimidazole suivant la revendication 1, caractérisé en ce que le radical $R_2$ est monosubstitué en la position para.

3. Le 1-[(2-phényl 2-éthyl) 2-N,N-diméthylaminoéthyl] imidazole et ses sels,

4. Le 1-[(1-phényl 1-éthyl) 2-N,N-diméthylaminoéthyl] imidazole et ses sels,

5. Le 1-[(1-phényl 1-éthyl) 2-N,N-diméthylaminoéthyl] 2-méthyl imidazole et ses sels.

6. Composition pharmaceutique, ayant notamment une activité cytoprotectrice et antisécrétoire gastrique, caractérisée en ce qu'elle comprend un composé suivant l'une des revendications précédentes associé à un véhicule thérapeutiquement administrable.

7. Procédé de préparation d'aminoéthylimidazole suivant la revendication 1, caractérisé en ce qu'il consiste à condenser un halogénure de 2-N,N' diméthylaminoéthyle de formule

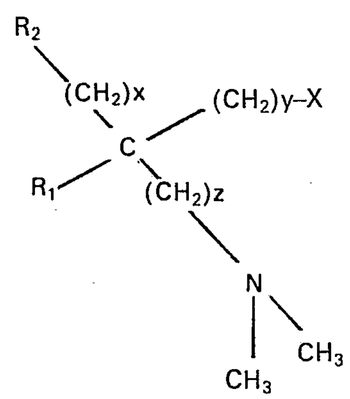

dans laquelle

$R_1$, $R_2$, x, y et z ont les significations énumérées à la revendication 1 et X est un halogène, notamment le chlore, sur un dérivé imidazolique de formule:

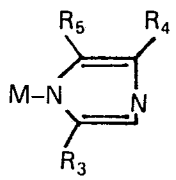

dans laquelle $R_3$, $R_4$ et $R_5$ ont les significations indiquées à la revendication 1 et M est un atome monovalent d'un métal alcalin ou l'argent et, pour préparer le sel, à salifier par un acide convenable, la base obtenue.

8. Procédé suivant la revendication 7, caractérisé en ce qu'il consiste à effectuer la condensation en milieu monophasique dans un solvant anhydre choisi parmi les hydrocarbures halogénés, les hydrocarbures aromatiques, les éthers, les éthers cycliques, les hydrocarbures aromatiques éventuellement alcoxylés, les sulfoxydes et les amides pendant 1 à 10 heures entre 20 et 150°C.

9. Procédé suivant la revendication 7, caractérisé en ce qu'il consiste à effectuer la condensation en milieu biphasique en présence d'eau, d'un hydrocarbure aromatique et d'un catalyseur de transfert de phase pendant 1 à 10 heures entre 20 et 150°C.

10. Procédé suivant la revendication 9, caractérisé en ce que le catalyseur est un dérivé d'ammonium quaternaire ou de phosphonium.

**Revendications pour l'Etat contractant AT**

1. Procédé de préparation d'aminoéthylimidazole de formule:

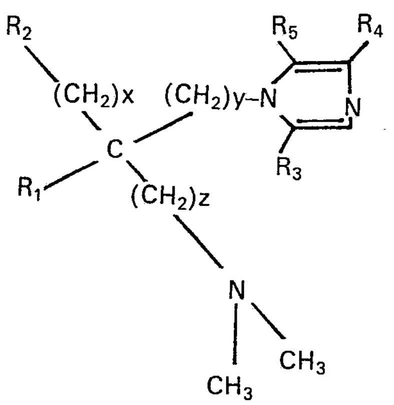

(I)

dans laquelle:

$R_1$ est alcoyle ayant jusqu'à six atomes de carbone $R_2$ est soit alcoyle ayant jusqu'à six atomes de carbone, soit un radical phényle ou phénoxy éventuellement substitué par un chlore, par un radical alcoxy ayant jusqu'à six atomes de carbone ou par un radical phényle.

$R_3$, $R_4$ et $R_5$ peuvent être identiques ou différents et sont des atomes d'hydrogène, des radicaux alcoyle ayant jusqu'à six atomes de carbone ou encore des radicaux phényle.

x, y et z ont pour valeur 0 ou 1, les valeurs de y et de z ne pouvant toutefois être égales, ou de leurs sels, caractérisé en ce qu'il consiste à condenser un halogénure de 2-N,N' diméthylaminoéthyle de formuel

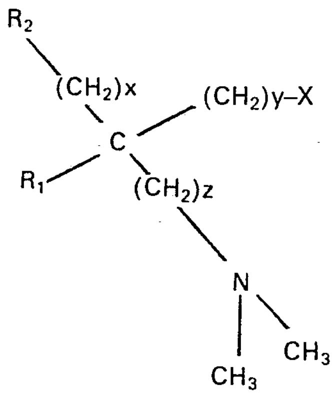

dans laquelle

X est un halogène, notamment le chlore, sur un dérivé imidazolique de formule:

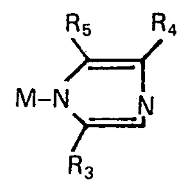

dans laquelle

M est un atome monovalent d'un métal alcalin ou l'argent et pour obtenir le sel, à salifier la base obtenue par un acide convenable.

2. Procédé suivant la revendication 1, caractérisé en ce qu'il consiste à effectuer la condensation en milieu monophasique dans un solvant anhydre choisi parmi les hydrocarbures halogénés, les hydrocarbures aromatiques, les éthers, les éthers cycliques, les hydrocarbures aromatiques éventuellement alcoxylés, les sulfoxydes et les amides pendant 1 à 10 heures entre 20 et 150°C.

3. Procédé suivant la revendication 2, caractérisé en ce qu'il consiste à effectuer la condensation en milieu biphasique en présence d'eau, d'un hydrocarbure aromatique et d'un catalyseur de transfert de phase pendant 1 à 10 heures entre 20 et 150°C.

4. Procédé suivant la revendication 3, caractérisé en ce que le catalyseur est un dérivé d'ammonium quaternaire ou de phosphonium.

5. Procédé suivant l'une des revendications précédentes pour la préparation d'un composé de formule I dans lequel le radical $R_2$ est monosubstitué en la position para.

6. Procédé suivant l'une des revendications 1 à 4 pour la préparation du 1-[(2-phényl 2-éthyl)-2-N,N-diméthylaminoéthyl] imidazole et de ses sels.

7. Procédé suivant l'une des revendications 1 à 4 pour la préparation du 1-[(1- phényl 1-éthyl) 2-N,N-diméthylaminoéthyl] imidazole et de ses sels.

8. Procédé suivant l'une des revendications 1 à 4 pour la préparation du 1-[(1-phényl 1-éthyl) 2-N,N-diméthylaminoéthyl] 2-méthyl imidazole et de ses sels.

9. Procédé de préparation d'une composition pharmaceutique, caractérisé en ce qu'il consiste à mélanger un principe actif comprenant au moins un composé de formule I tel que défini à la revendication 1 ou l'un de ses sels acceptables thérapeutiquement à un véhicule pharmaceutique.

10. L'utilisation d'un composé de formule I tel que défini à la revendication 1 ou de l'un de ses sels acceptable thérapeutiquement pour l'obtention d'un médicament destiné à une utilisation cytoprotectrice.

**Patentansprüche für die Vertragsstaaten BE, CH, DE, FR, GB, IT, LI, LU, NL, SE**

1. Aminoethylimidazol der Formel:

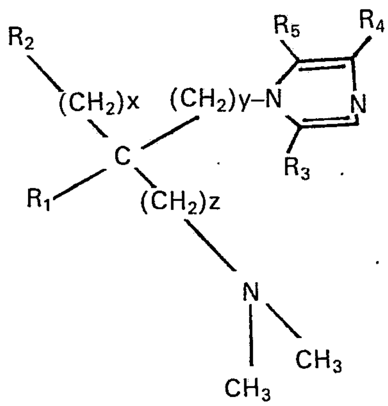

(I)

worin bedeuten:

$R_1$ eine Alkylgruppe mit bis zu 6 Kohlenstoffatomen;

$R_2$ eine Alkylgruppe mit bis zu 6 Kohlenstoffatomen, einen Phenyl- oder Phenoxyrest, gegebenenfalls durch ein Chloratom, einen Alkylrest mit bis zu 6 Kohlenstoffatomen oder einen Phenylrest, substituiert;

$R_3$, $R_4$ und $R_5$ können gleich oder verschieden sein und bedeuten Wasserstoff, Alkylreste mit bis zu 6 Kohlenstoffatomen oder einen Phenylrest;

x, y und z entsprechen dem Wert 0 oder 1, wobei die Werte von y und z jedoch nicht gleich sind und die Säureadditionssalze.

2. Aminoethylimidazol nach Anspruch 1, dadurch gekennzeichnet, dass der Rest $R_2$ in para-Stellung monosubstituiert ist.

3. 1-[(2-Phenyl-2-ethyl)-2-N,N-dimethylaminoethyl]-imidazol und seine Salze.

4. 1-[(1-Phenyl-1-ethyl) -2-N,N-dimethylaminoethyl]-imidazol und seine Salze.

5. 1-[(1-Phenyl-1-ethyl)- 2-N,N-dimethylaminoethyl]- 2-methyl- imidazole und seine Salze.

6. Pharmazeutische Zusammensetzung mit einer insbesondere cytoprotektiven und antisekretiven Aktivität im Magen, dadurch gekennzeichnet, dass sie eine Verbindung nach einem der vorhergehenden Ansprüche in Verbindung mit einem therapeutisch annehmbaren Trägermaterial enthält.

7. Verfahren zur Herstellung von Aminoethylimidazol nach Anspruch 1, dadurch gekennzeichnet, dass man ein Halogenid von 2-N,N'-Dimethylaminoethyl der Formel

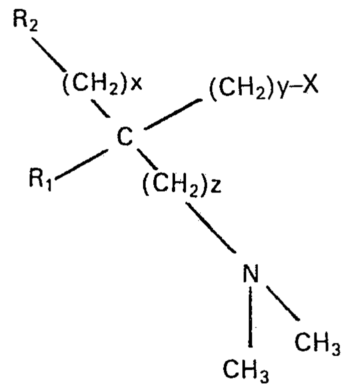

worin

$R_1$, $R_2$, x, y und z die in Anspruch 1 gegebene Bedeutung aufweisen, und X ein Halogenatom, insbesondere Chlor bedeutet, mit einem Imidazolderivat der Formel:

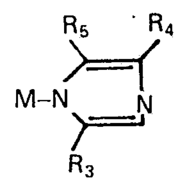

umsetzt, worin

$R_3$, $R_4$ und $R_5$ die in Anspruch 1 gegebene Bedeutung aufweisen und M ein monovalentes Alkalimetallatom oder Silber ist, und zur Herstellung des Salzes die erhaltene Base durch eine geeignete Säure in das Salz überführt.

8. Verfahren nach Anspruch 7, dadurch gekennzeichnet, dass man die Umsetzung im einphasischen Milieu in einem wasserfreien Lösungsmittel, ausgewählt aus Halogenkohlenwasserstoffen, aromatischen Kohlenwasserstoffen, Ether, cyclische Ether, gegebenenfalls alkoxylierte aromatische Kohlenwasserstoffe, Sulfoxide und Amide, während 1 bis 10 Stunden zwischen 20 und 150°C durchführt.

9. Verfahren nach Anspruch 7, dadurch gekennzeichnet, dass man die Umsetzung in einem biphasischen Milieu in Gegenwart von Wasser, eines aromatischen Kohlenwasserstoffs und eines Phasentransferkatalysators während 1 bis 10 Stunden zwischen 20 und 150°C durchführt.

10. Verfahren nach Anspruch 9, dadurch gekennzeichnet, dass der Katalysator ein quaternäres Ammonium- oder Phosphoniumderivat ist.

**Patentansprüche für den Vertragsstaat AT**

1. Verfahren zur Herstellung von Aminoethylimidazol der Formel

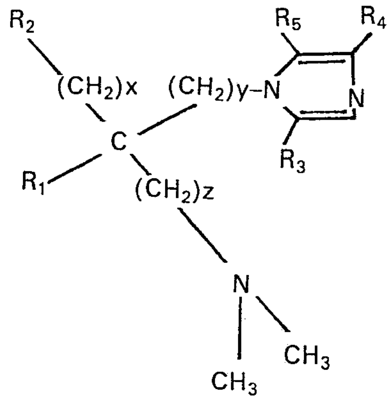

(I)

worin bedeuten:

$R_1$ eine Alkylgruppe mit bis zu 6 Kohlenstoffatomen;

$R_2$ eine Alkylgruppe mit bis zu 6 Kohlenstoffatomen, einen Phenyl- oder Phenoxyrest, gegebenenfalls durch ein Chloratom, einen Alkylrest mit bis zu 6 Kohlenstoffatomen oder einen Phenylrest, substituiert;

$R_3$, $R_4$ und $R_5$ können gleich oder verschieden sein und bedeuten Wasserstoff, Alkylreste mit bis zu 6 Kohlenstoffatomen oder einen Phenylrest;

x, y und z entsprechen dem Wert 0 oder 1, wobei die Werte von y und z jedoch nicht gleich sind und die Säureadditionssalze, dadurch gekennzeichnet, dass man ein Halogenid von 2-N,N'-Dimethylaminoethyl der Formel

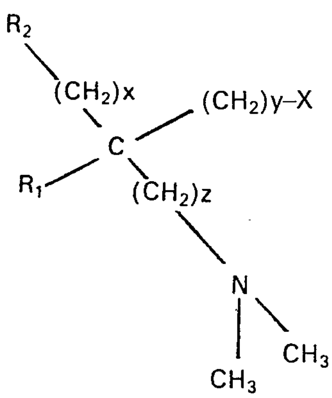

worin bedeuten:

X ein Halogenatom, insbesondere Chlor, mit einem Imidazolderivat der Formel:

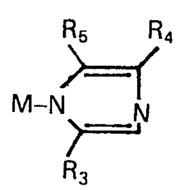

worin bedeuten M ein monovalentes Alkalimetallatom oder Silber, umsetzt, und um das Salz zu erhalten die erhaltene Base mit einer geeigneten Säure in das Salz überführt.

2. Verfahren nach Anspruch 1, dadurch gekennzeichnet, dass man die Umsetzung im einphasischen Milieu in einem wasserfreien Lösungsmittel, ausgewählt aus Halogenkohlenwasserstoffen, aromatischen Kohlenwasserstoffen, Ether, cyclische Ether, gegebenenfalls alkoxylierte aromatische Kohlenwasserstoffe, Sulfoxide und Amide während 1 bis 10 Stunden zwischen 20 und 150°C durchführt.

3. Verfahren nach Anspruch 2, dadurch gekennzeichnet, dass man die Umsetzung in einem biphasischen Milieu in Gegenwart von Wasser, eines aromatischen Kohlenwasserstoffs und eines Phasentransferkatalysators während 1 bis 10 Stunden zwischen 20 und 150°C durchführt.

4. Verfahren nach Anspruch 3, dadurch gekennzeichnet, dass der Katalysator ein quaternäres Ammonium- oder Phosphoniumderivat ist.

5. Verfahren nach einem der vorhergehenden Ansprüche zur Herstellung einer Verbindung der Formel I, worin der Rest $R_2$ in der para-Stellung monosubstituiert ist.

6. Verfahren nach einem der Ansprüche 1 bis 4 zur Herstellung von 1-[(2-Phenyl-2-ethyl)-2-N,N-dimethylaminoethyl]- imidazol und seiner Salze.

7. Verfahren nach einem der Ansprüche 1 bis 4 zur Herstellung von 1-[(1-Phenyl-1-ethyl)-2-N,N-dimethylaminoethyl] -imidazol und seiner Salze.

8. Verfahren nach einem der Ansprüche 1 bis 4 zur Herstellung von 1-[(1-Phenyl-1-ethyl) -2-N,N-dimethylaminoethyl]-2- methylimidazol und seiner Salze.

9. Verfahren zur Herstellung einer pharmazeutischen Zusammensetzung, dadurch gekennzeichnet, dass man einen aktiven Bestandteil der mindestens eine Verbindung der Formel I gemäss Anspruch 1 oder ein therapeutisch annehmbares Salz enthält, mit einem pharmazeutischen Trägermaterial vermischt.

10. Verwendung einer Verbindung der Formel I gemäss Anspruch 1 oder eines therapeutisch annehmbaren Salzes zur Herstellung eines Medikaments zur Verwendung als cytoprotektives Mittel.

**Claims for the Contracting States BE, CH, DE, FR, GB, IT, LI, LU, NL, SE**

1. Aminoethyl imidazole of formula:

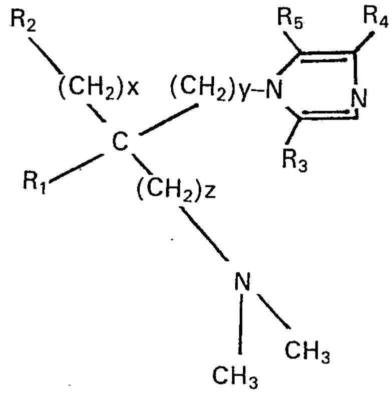

(I)

in which:

$R_1$ is alkyl having up to six carbon atoms

$R_2$ is either alkyl having up to six carbon atoms or a phenyl or phenoxy radical optionally substituted by a chlorine Atom, an alkoxy radical having up to six carbon atoms or a phenyl radical, $R_3$, $R_4$ and $R_5$, which can be the same or different, are hydrogen atoms, alkyl radicals having up to six carbon atoms or phenyl radicals, whilst x, y and z have the values 0 or 1, but the values of y and z cannot be the same, together with their addition salts with acids.

2. Aminoethyl imidazole according to claim 1, wherein the $R_2$ radical is monosubstituted in the para-position.

3. 1-[(2-phenyl-2-ethyl)-2-N,N-dimethylaminoethyl]- imidazole and its salts.

4. 1-[(1-phenyl-1-ethyl)-2-N,N-dimethylaminoethyl]-imidazole and its salts.

5. 1-[(1-phenyl-1-ethyl)- 2-N,N-dimethylaminoethyl]-2- methyl-imidazole and its salts.

6. A pharmaceutical composition more particularly having a cytoprotective and gastric antisecretory activity, characterized in that it comprises a compound according to anyone of the preceeding claims together with a therapeutically administrable carrier.

7. A process for the preparation of the aminoethyl imidazole of claim 1, characterized in that it comprises condensing a halide of 2-N,N'-dimethylaminoethyl of formula

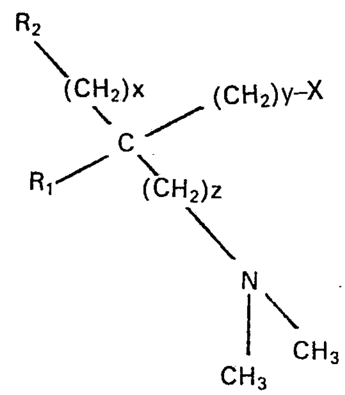

in which $R_1$, $R_2$, x, y and z have the meanings mentioned in claim 1 and X is a halogen, particularlychlorine, with an imidazole derivative of formula

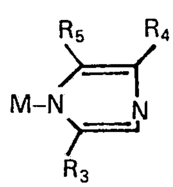

in which $R_3$, $R_4$ and $R_5$ have the meanings mentioned in claim 1 and M is a monovalent atom of an alkali metal or silver and, for preparing the salt, saiifying the prepared base with an appropriated acid.

8. A process according to claim 7, characterized in that it comprises performing the condensation in a monophase medium in an anhydrous solvent chosen from among halogenated hydrocarbons, aromatic hydrocarbides, ethers, cyclic ethers, optionally alkoxylated aromatic hydrocarbons, sulphoxides and amides for 1 to 10 hours at between 20 and 150°C.

9. A process according to claim 7, characterized in that it comprises carrying out the condensation in a two-phase medium in the presence of water, an aromatic hydrocarbon and a phase transfer catalyst for 1 to 10 hours at between 20 and 150°C.

10. A process according to claim 9, characterized in that the catalyst is a phosphonium or quaternary ammonium derivative.

**Claims for the Contracting State AT**

1. A process for the preparation of aminoethyl imidazole of formula:

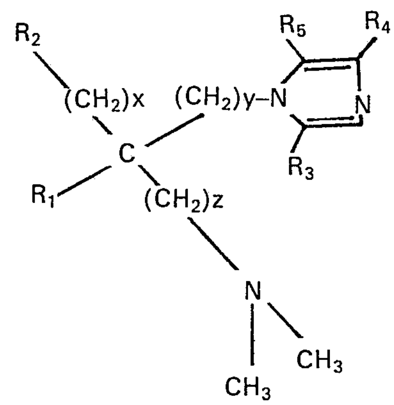

(I)

in which:

$R_1$ is alkyl having up to six carbon atoms

$R_2$ is either alkyl having up to six carbon atoms or a phenyl or phenoxy radical optionally substituted by a chlorine atom, an alkoxy radical having up to six carbon atoms or a phenyl radical, $R_3$, $R_4$ and $R_5$, which can be the same or different, are hydrogen atoms, alkyl radicals having up to six carbon atoms or phenyl radicals, whilst x, y and z have the values 0 or 1, but the values of y and z cannot be the same and their addition salts with acides, characterized in that it comprises condensing a halide of 2-N,N'-dimethylaminoethyl of formula

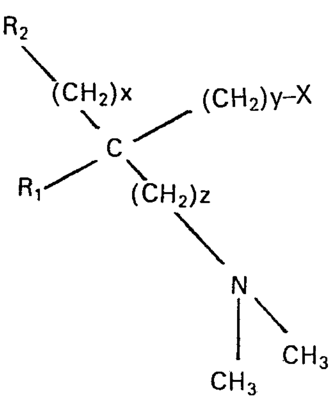

in which X is a halogen, particularly chlorine, with an imidazole derivative of formula

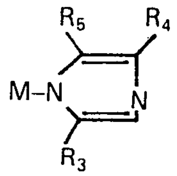

M being a monovalent atom of an alkali metal or silver, and for preparing the salt salifying the prepared base with an appropriate acid.

2. A process according to claim 1, characterized in that it comprises performing the condensation in a monophase medium in an anhydrous solvent chosen from among halogenated hydrocarbons, aromatic hydrocarbides, ethers, cyclic ethers, optionally alkoxylated aromatic hydrocarbons, sulphoxides and amides for 1 to 10 hours at between 20 and 150°C.

3. A process according to claim 2, characterized in that it comprises carrying out the condensation in a two-phase medium in the presence of water, an aromatic hydrocarbon and a phase transfer catalyst for 1 to 10 hours at between 20 and 150°C.

4. A process according to claims 3, characterized in that the catalyst is a phosphonium or quaternary ammonium derivative.

5. A process according to anyone of the preceeding claims for the preparation of a compound of formula I in which the $R_2$ radical is monosubstituted in the para-position.

6. A process according to claims 1 to 4 for preparing 1-[(2- phenyl-2-ethyl)-2-N,N-dimethylaminoethyl]- imidazole and its salts.

7. A process according to claims 1 to 4 for preparing 1-[(1- phenyl-1-ethyl)- 2-N,N-dimethylaminoethyl]-imidazole and its salts.

8. A process according to claims 1 to 4 for preparing 1-[(1-phenyl- 1-ethyl)-2N,N-dimethylaminoethyl]- 2-methyl-imidazole and its salts.

9. A process for preparing a pharmaceutical composition, characterized in that it comprises mixing a compound of formula I as defined in claim 1 or a therapeutically acceptable salt thereof with a pharmaceutically carrier.

10. The use of a compound of formula I as defined in claim 1 or of a therapeutically acceptable salt thereof for preparing a drug having a cytoprotective use.